# FASCICULE DE BREVET EUROPEEN

(11) **EP 2 385 813 B1**
(45) Date de publication et mention de la délivrance du brevet: **25.02.2015**
(21) Numéro de dépôt: 10704380.4
(22) Date de dépôt: 08.01.2010
(51) Int. Cl.: A61F 2/30, A61F 2/42

(54) **IMPLANT ORTHOPEDIQUE POUR ARTHROPLASTIE DIGITALE**
ORTHOPÄDISCHES IMPLANTAT FÜR ARTHOPLASTIE DER FINGER
ORTHOPAEDIC IMPLANT FOR ARTHROPLASTY OF THE FINGERS

(30) Priorité: 08.01.2009 FR 0900054
(43) Date de publication de la demande: 16.11.2011
(73) Titulaire: Memometal Technologies, 35170 Bruz (FR)
(72) Inventeur: BELLEMERE, Philippe, F-44000 Nantes (FR); DREANO, Thierry, F-35000 Rennes (FR); GOUBAU, Jean, B-8210 Loppem (BE); MARTINACHE, Xavier, F-51000 Reims (FR); PAPALOIZOS, Michaël, CH-1204 Genève (CH); PRANDI, Bernard, F-35700 Rennes (FR); SIRET, Pierre, F-35160 Talensac (FR); TCHURUKDICHIAN, Alain, F-21000 Dijon (FR)
(74) Mandataire: Benech, Frédéric
(86) Numéro de dépôt international: PCT/FR2010/000010
(87) Numéro de publication internationale: WO 2010/079288

(56) Documents cités:
- WO-A-02/43627
- FR-A- 2 734 150
- FR-A- 2 743 717
- US-A- 4 242 759
- US-A- 4 725 280

## Description

La présente invention concerne un implant orthopédique utilisé en arthroplastie digitale comprenant un premier élément pour phalange proximale et un second élément pour phalange adjacente distale.

Par phalange proximale on entend une phalange située du côté du corps ou du membre (main ou pied) concerné, et par phalange distale une phalange située vers l'extérieur de ce membre, selon les conventions d'orientation adoptée classiquement en anatomie, et qui seront par ailleurs utilisées ci-après.

Elle concerne également un procédé de mise en place d'un tel implant.

Elle trouve une application particulièrement importante bien que non exclusive dans le domaine médical des prothèses de l'articulation Inter-Phalangienne Proximale de la main (connue dans le domaine par ses initiales IPP).

Mais elle peut également et notamment être avantageusement utilisée pour des prothèses articulaires digitales Inter-Phalangiennes du pouce (IP pouce)ou pour des prothèses Inter-Phalangiennes Distales (IPD), pour la main ou pour le pied, et ce par simple adaptation géométrique aux sites osseux concernés.

On connaît déjà des prothèses d'articulation inter-phalangiennes comprenant des premiers et des seconds éléments comportant chacun respectivement une tige d'implantation dans l'os et une tête d'articulation inter-phalangienne complémentaire.

Par exemple il est connu (EP 1 339 362) un implant comportant un premier élément muni d'une tête présentant une surface convexe d'articulation bi-condylienne comportant une vallée centrale et un second élément muni d'une tête complémentaire présentant une surface biconcave d'articulation agencée pour coopérer avec la surface convexe de sorte qu'il y ait une congruence entre ces deux surfaces dans le plan sagittal.

Par congruence, on entend le fait de coïncider, de s'ajuster parfaitement dans le plan sagittal.

La congruence dans le plan sagittal signifie donc que le point de contact entre les deux surfaces articulaires dans ce plan parcourt toujours la même ligne de contact surfacique lors du mouvement.

Rappelons qu'un plan sagittal ou para-sagittal est un plan parallèle au plan médian lorsque le corps humain est dans la position de Poirier.

Une telle disposition, si elle permet un bon guidage de l'articulation, se révèle à force préjudiciable à la pérennité de la prothèse. Elle peut en effet entraîner des blocages et donc un échec de la prothèse jusqu'à dans 20% des cas, en fonction du chirurgien qui opère ; elle génère de plus une usure importante des surfaces articulaires de la prothèse nécessitant de réopérer.

On connaît également (EP 0 572 339), des prothèses comportant des surfaces bi-condyliennes à rayons de courbure distincts, en forme de tonneau.

Ici encore ce type d'implant, surtout utilisé dans le cadre de prothèse d'épaule, pose des problèmes d'usure et se révèle par ailleurs mal adapté aux articulations de petites dimensions comme celles des doigts.

Pour tenter de palier ces inconvénients, il a été envisagé (DE 198 20748) des prothèses en bio céramique, pour doigt de main ou doigt de pied.

Si de telles prothèses résistent mieux à l'usure, elles présentent cependant des risques d'éclatement et sont de plus complexes à mettre en place. Un implant orthopédique selon les caractéristiques du préambule de la revendication 1 est connu du document FR-A-2743717.

La présente invention vise à fournir un implant orthopédique et un procédé répondant mieux que ceux antérieurement connus aux exigences de la pratique notamment en ce qu'elle engendre une usure très faible malgré de nombreux mouvements d'articulation, et en ce qu'elle présente une grande facilité de montage en s'adaptant aux contraintes liées aux fonctionnements ultérieurs des articulations arthroplastiques.

Elle présente de plus un coût faible et permet une grande modularité, c'est à dire une grande diversité dans les tailles.

Dans ce but la présente invention propose un implant orthopédique utilisé en arthroplastie digitale comprenant un premier élément pour phalange proximale et un second élément pour phalange adjacente distale, chaque élément comportant respectivement une tige d'implantation dans l'os et une tête d'articulation inter-phalangienne, la tête du premier élément présentant une surface d'articulation condylienne, bi-convexe, comportant une vallée centrale, et la tête du second élément présentant une surface d'articulation bi-concave agencée pour coopérer avec ladite surface bi-convexe et comportant une crête centrale, caractérisé en ce que les surfaces au repos sont congruentes dans le plan frontal de l'articulation au niveau des condyles et non congruentes dans le plan sagittal.

Par au repos il faut entendre une absence de pression externe sur les surfaces de l'articulation.

La congruence dans le plan frontal au niveau des condyles signifie que la zone de contact entre les deux surfaces articulaires est matérialisée par deux lignes horizontales qui balayent verticalement la surface lors du mouvement.

De façon étonnante cette congruence dans le plan frontal associée à une non congruence dans le plan sagittal, assure en présence de liquide articulaire, une excellente lubrification de l'articulation pendant le mouvement et limite ainsi considérablement l'usure des surfaces articulaires, et l'enraidissemnt.

Dans des modes de réalisation avantageux, on a de plus recours à l'une et/ou à l'autre des dispositions suivantes :
- les rayons de courbure (R1, R'1) des condyles de la surface bi-convexe du premier élément sont respectivement identiques aux rayons de courbure (R2, R'2) de la surface bi-concave correspondante du second élément dans le plan frontal, le rayon de courbure (R3) de la surface proximale du premier élément étant inférieur au rayon de courbure (R4) de la surface distale correspondante du second élément dans le plan sagittal.

Par rayon de courbure dans le plan frontal on entend le rayon de la courbe formée par l'intersection de la surface d'articulation avec le plan transversal orthogonal à ladite surface de la tête ;
- les rayons de courbures sont tels que R1 = R'1 = R2 = R'2 ;
- la matière des têtes est agencée pour permettre aux dites têtes de coopérer à frottement sur une ligne de frottement dans le plan frontal en l'absence de pression longitudinale et sur une surface d'écrasement dans ledit plan frontal en cas de pression longitudinale.

Par pression longitudinale on entend une pression exercée dans le sens longitudinal d'une ou des deux phalanges concernées. Autrement dit, cette disposition permet notamment d'obtenir une congruence maximale sous charge, transformant les deux lignes de contact précédemment évoquées en une bande de contact qui répartit ainsi les efforts et diminue encore de façon surprenante l'usure des surfaces articulaires ;
- la matière de la surface bi-convexe de la tête du premier élément est un matériau polymère souple, dont le module d'Young E ≤ 30 Gpa.

Ainsi les deux surfaces articulaires sont de rigidités différentes, le matériau le plus souple et d'usure étant du coté condyle.

On notera à ce sujet que la surface condylienne étant en général et par construction plus épaisse que la surface distale, ceci permet une moindre déformation du matériau souple.

Par ailleurs, la surface distale balayant une grande partie de la surface condylienne, entre la position droite et la position fléchie, il y a ainsi une meilleure répartition de l'usure.

On obtient ainsi un compromis entre une élasticité très grande proche de celle du cartilage anatomique et une rigidité plus forte pour limiter la déformation et l'usure à long terme.

Avantageusement il peut également s'agir du matériau connu sous la dénomination CFR PEEK, initiales anglo-saxonnes pour Carbon Fiber Reinforced PolyEtherEtherKetone de module d'Young de l'ordre de 18 Gpa ;
- le module d'Young de la surface bi-condylienne est E ≤ 2 Gpa. Plus précisément et de façon non limitative, le matériau souple est un polyéthylène connu sous la dénomination UHMWPE (initiales anglo-saxonnes de Ultra Hight Molecular Wight Poly Ethylen) avec E ≤ 1 Gpa ;
- les surfaces respectives des têtes des deux éléments placées en contact en vis à vis, définissent un espace inter-condylien de volume non nul sur toute la longueur de la vallée centrale, permettant une lubrification améliorée de l'articulation entre les têtes.
   Un tel espace inter-condylien par exemple compris entre 15 mm³ et 30 mm³, par exemple de 25 mm³ permet d'améliorer encore la lubrification d'articulation, le contact entre les surfaces articulaires à cet endroit étant donc non congruent ;
- la vallée de la tête bi-condylienne du premier élément présente dans le plan frontal un rayon de courbure R5, la crête de la surface biconcave du second élément présentant quant-à-elle un rayon de courbure R6 > R5 ;
- les tiges d'implantation ont une forme de pieu polygonal proportionné pour être reçu à l'intérieur de la cavité médullaire d'une phalange et sont munies d'une butée d'extrémité anti-enfoncement.

Avantageusement le matériau et la forme de la tige présentent un module d'élasticité ou module d'Young effectif E ≤ 30 GPa.

Par module d'Young effectif ou apparent on entend un module d'Young global de la tige dont le comportement est identique à celui d'un matériau ayant intrinsèquement ce module d'Young.

Plus précisément, on peut prévoir deux raideurs apparentes de tiges différentes selon les patients, soit une tige rigide pour les patients jeunes (i.e. de l'ordre de 20 GPa) et une tige plus souple pour les patients âgés (i.e. de l'ordre de 5 à 6 GPa) ;
- au moins une tige d'implantation présente une forme au moins en partie ajourée dans le plan dorsopalmaire ;
- au moins une tige d'implantation présente une section transversale en H, en U ou en V, et/ou présente une fente en bec de canard, une forme évidée en demi-cylindre côté palmaire ou en demi-tube.

Avantageusement les tiges sont ajourées et/ou sont recouvertes d'un revêtement ostéo-intégrateur, total ou partiel, ce qui améliore l'ancrage ;
- les tiges d'implantation des éléments étant amovibles par rapport aux têtes correspondantes, elles comportent des moyens de connexion avec lesdites têtes ;
- les moyens de connexion sont formés par un emmanchement conique d'un pion solidaire de la tête dans un évidemment ménagé dans la tige correspondante ;
- l'emmanchement conique est un cône Morse, c'est à dire avec un angle de conicité de l'ordre de 5 % : mais cette conicité peut également être plus faible, par exemple de l'ordre de 3 % ou 2,5 % ;
- la surface biconcave comporte une base munie du pion de connexion, d'épaisseur déterminée.
   Le fait de prévoir une base d'épaisseur déterminée, permet ainsi plusieurs épaisseurs déterminées, que le chirurgien va pouvoir choisir et adapter à la coupe effectuée lors de l'opération ;
- les moyens de connexion entre les parties d'un élément formées par la tige et la tête correspondante comprennent de plus un évidemment ou une rainure formé dans l'une des parties propre à recevoir une plaquette de forme complémentaire solidaire de l'autre partie ;
- la surface bi-convexe d'articulation bi-condylienne et la surface bi-concave correspondante présentent des rayons de courbure uniformes dans le plan frontal et des rayons de courbure variables dans le plan sagittal ;
- les moyens de connexion sont identiques entre d'une part, plusieurs têtes de tailles différentes et d'autre part, des tiges identiques ou différentes de premier éléments et/ou de second éléments.

Une telle disposition autorise une grande modularité.

L'invention propose également un procédé de mise en place d'un implant orthopédique pour arthroplastie digitale du type décrit ci-dessus.

L'invention sera mieux comprise à la lecture de la description qui suit de modes de réalisation donnés ci-après à titre d'exemples non limitatifs.

La description se réfère aux dessins qui l'accompagnent dans lesquels :
La figure 1 est une vue schématique de dessus, d'un squelette de main dans lequel un implant selon l'invention a été mis en place.
La figure 2 montre en perspective la tête bi-convexe d'un premier élément selon un mode de réalisation de l'invention.
La figure 3 est une vue en perspective d'un second élément comprenant une tête bi-concave agencée pour coopérer avec la tête du premier élément de la figure 2.
La figure 4 est une vue schématique de dessus ou dorsale montrant un implant selon l'invention, articulation allongée.
La figure 5 est une vue latérale en coupe de l'implant, côté palmaire en haut, de la figure 4.
Les figures 6 et 7 sont respectivement des vues en coupes selon VI-VI et selon VII-VII, des figures 4 et 5, montrant les rayons de courbure respectivement dans les plans sagittal et frontal, des surfaces d'articulation selon le mode de réalisation de l'invention plus particulièrement décrit ici.
La figure 8 montre la tête de la figure 2 sous contrainte longitudinale, faisant apparaître une surface d'écrasement dans le cas de l'utilisation d'un matériau de tête plus souple selon un mode de réalisation de l'invention.
Les figures 8A et 8B sont des schémas en coupe de l'articulation respectivement en position doigt fléchi, et doigt droit, montrant la zone de contact d'écrasement entre les surfaces.
La figure 9 montre une tige d'implant utilisable avec la tête de la figure 8, selon un mode de réalisation de l'invention.
La figure 10 est une vue en coupe latérale d'un élément d'implant comprenant une tige et une tête selon un mode de réalisation de l'invention.
La figure 11 montre schématiquement, en vue latérale trois modes de réalisation de tête pour second élément selon l'invention, avec des bases d'épaisseurs différentes.
Les figures 12 à 16 montrent en perspective arrière, cinq modes de réalisation de tige pour implantation selon l'invention.

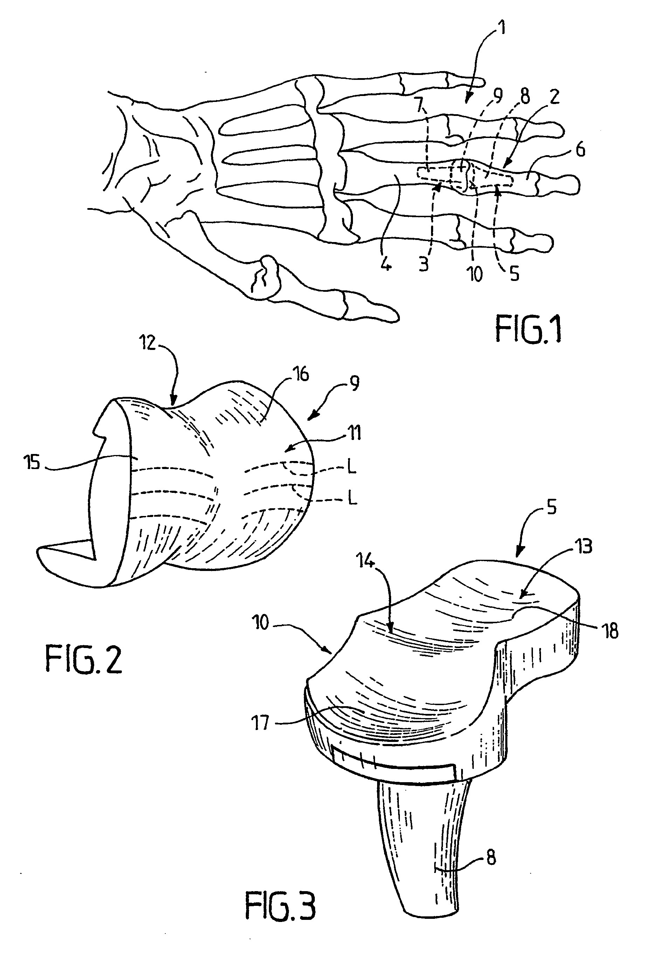

La figure 1 est une vue schématique de dessus d'une main 1 de squelette, comportant un implant orthopédique 2 selon l'invention.

Cet implant comprend un premier élément 3 pour phalange proximale 4 et un second élément 5 pour phalange distale 6.

Chaque élément comporte respectivement une tige d'implantation dans l'os 7, 8 et une tête d'articulation 9, 10 inter-phalangienne.

On utilisera par la suite les mêmes numéros de référence pour désigner des éléments semblables ou identiques.

La figure 2 montre la tête 9 du premier élément 3.

Elle présente une surface 11 d'articulation condylienne bi-convexe comportant une vallée centrale 12.

La figure 3 montre quant-à-elle le second élément 5 comprenant une tige d'implantation 8 et la tête 10.

Celle-ci présente une surface bi-concave 13 agencée pour coopérer avec la surface bi-convexe 11 de la tête du premier élément.

Cette surface bi-concave 13 comporte une crête centrale 14.

Les surfaces 11 et 13 sont agencées pour être congruentes au niveau des deux condyles 15 et 16 et des surfaces concaves correspondantes 17 et 18 dans le plan frontal de l'articulation et non congruentes dans le plan sagittal.

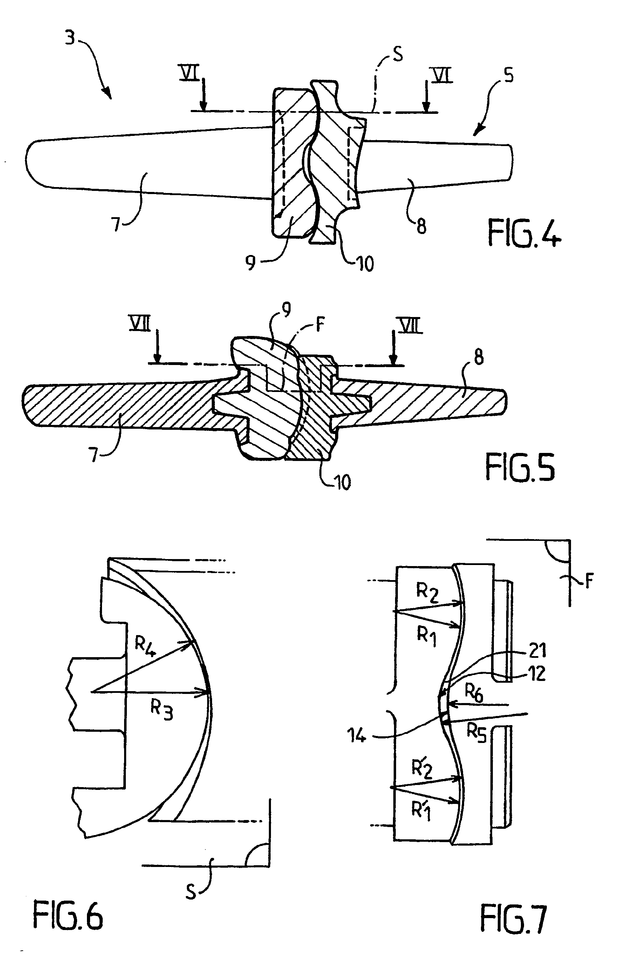

Plus précisément on a représenté sur les figures 4, 5, 6 et 7 le mode de réalisation plus particulièrement décrit ici de cette congruence ou de cette non congruence.

Pour ce faire les surfaces des condyles et les surfaces en vis à vis comportent des rayons de courbure tels que en référence à la figure 7 dans le plan frontal F, les rayons de courbure R1, R'1 des condyles de surface convexe sont identiques ou sensiblement identiques aux rayons de courbure R2, R'2 des surfaces correspondantes concaves du second élément.

Par sensiblement identique on entend une valeur égale à ± 5%.

Par contre, le rayon de courbure R3 de la surface proximale du premier élément (cf. figure 6) est inférieur au rayon de courbure R4 de la surface distale correspondante du second élément dans le plan sagittal S, par exemple compris entre 1,1 et 1,5 fois, par exemple 1,2 fois inférieur.

Ceci permet d'obtenir des congruences et des non congruences telles que mentionnées ci-dessus.

Par exemple R3 est compris entre 3,4 mm (taille S), 3,75 mm (taille M) et 4,5 mm (taille L) pour un rayon R4 = 5 mm.

Avantageusement, les rayons R1, R'1, R2, R'2 sont tous égaux par exemple à une valeur comprise entre 4mm et 6mm, par exemple 5mm.

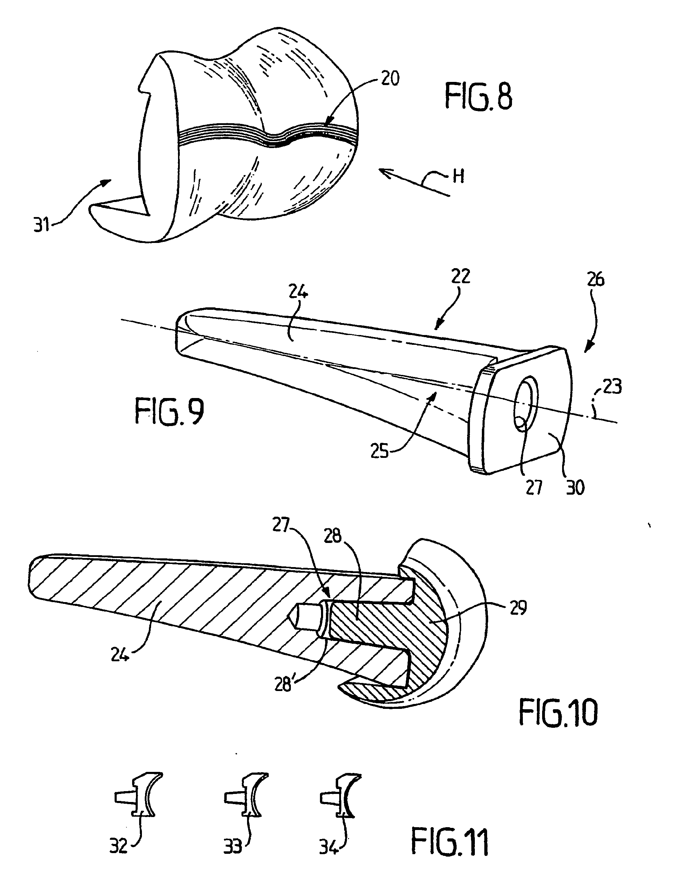

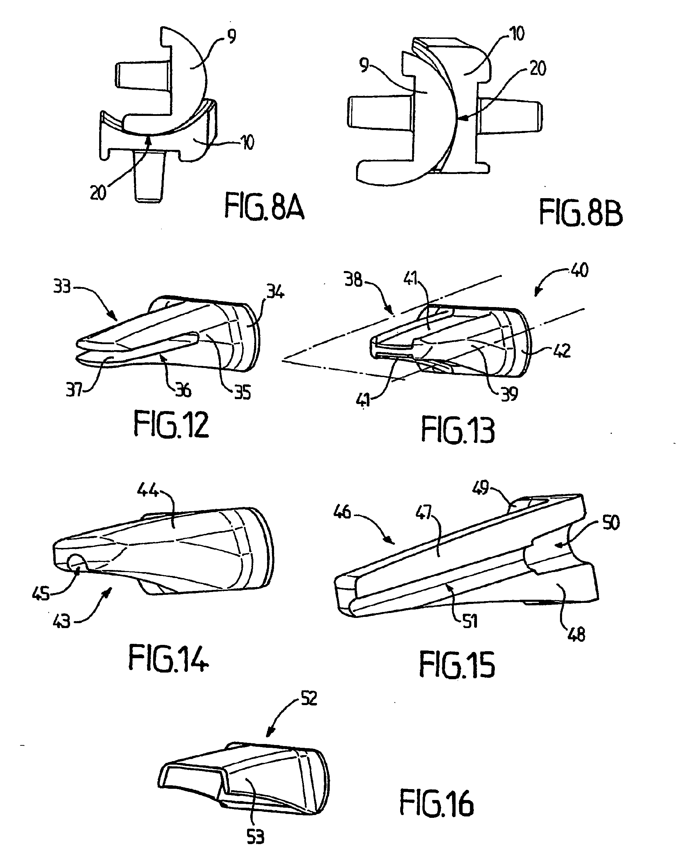

On a représenté sur la figure 8 la surface de contact 20 obtenue avec une tête de premier élément constituée en matériau élastique lorsqu'une pression longitudinale (flèche H de la figure 8) est exercée.

La ligne de frottement dans le plan frontal se transforme en effet et alors en une surface 20, ce qui permet de mieux répartir les efforts de friction, et de minimiser ainsi de façon inattendue l'usure à terme de la tête.

En d'autres termes, et grâce à la combinaison du matériau de cette tête, qui peut par exemple être un polymère type UHMWPE, et des rayons de courbure respectifs mentionnés ci-dessus, on obtient ainsi un excellent amortissement et une usure minimisée.

Toujours en référence aux figures 6 et 7, il est prévu un espace inter-condylien 21, de volume non nul sur toute la longueur de la vallée centrale 12 en vis à vis de la crête 14, espace qui facilite la lubrification pour le liquide, par exemple un volume théorique qui va dépendre de la pression sur la prothèse de l'ordre de 20 mm³ , par exemple 24 mm³.

Pour ce faire, le rayon de courbure R5 de la vallée et le rayon de courbure R6 de la crête sont tels que R6 est supérieur à R5, par exemple pour l'un de 4,5 mm et pour l'autre de 2,5 mm.

Selon l'invention plus particulièrement décrite ici, l'implant comporte deux éléments 3 et 5, chacun muni d'une tête 9, 10 et d'une tige correspondante amovible 7, 8 désolidarisable de la tête, de fixation dans l'os.

On a représenté sur les figures 8A et 8B l'évolution de la zone de contact 20 au cours du mouvement, doigt fléchi (figure 8A) et doigt droit (figure 8B).

On constate que la zone de contact 20 balaye la moitié du condyle mais reste à peu près au même endroit sur la partie distale. La surface la plus fragile et ainsi choisie coté condyle selon le mode de réalisation de l'invention plus particulièrement décrit ici.

On a représenté sur la figure 9 un exemple de telle tige d'implantation 22 en titane.

La tige 22 comporte une partie allongée sensiblement autour d'un axe 23, légèrement courbe, par exemple avec un rayon de courbure p de 100 mm pour une tige pour élément proximal de grande taille et de 90 mm pour élément proximal de moyenne ou petite taille ou encore compris entre 50 mm (grande taille), 40 mm (taille moyenne) et 10 mm (petite taille) pour les éléments distals.

La tige est munie d'une partie agencée pour être introduite dans le trou central de la moelle épinière de la phalange, à savoir la partie 24 de forme sensiblement polygonale, par exemple hexagonale avec une partie légèrement tronconique d'extrémité 25 permettant une bonne introduction et un blocage latéral en indexation dans la cavité modulaire de la phalange.

La tige d'implantation est montée de façon amovible par rapport à la tête et comporte des moyens 26 de connexion avec ladite tête.

Ces moyens de connexion comportent un orifice (cf. également figure 10) conique 27 dans lequel vient s'emmancher un pion conique 28 solidaire de la tête 29, par exemple présentant un angle de conicité de 2 à 3 degré.

La tige comporte de plus une plaquette 30 anti-enfoncement qui vient coopérer avec un évidemment complémentaire en forme de rainure 31 (voir figure 8) ce qui permet lors de l'enfoncement du pion 28 dans l'évidemment 28' et blocage de la plaquette 30 dans l'évidemment 31, une bonne solidarisation et une bonne indexation de la tête 29 avec la tige 24.

Du côté du second élément, avantageusement la tête 10 comporte une base (cf. figure 11) 32, 33, 34 d'épaisseur variable ce qui permet d'ajuster au moment du montage de l'implant les épaisseurs pour optimiser le fonctionnement de l'articulation lors de l'opération chirurgicale.

Cette base peut par exemple être d'une épaisseur de 1, 2 ; 1,7 ou 2,2 mm.

Avantageusement la surface convexe d'articulation bi-condylienne et la surface bi-concave présente des rayons de courbure uniformes dans le plan frontal et différents dans le plan sagittal.

Le fait d'uniformiser et de standardiser les dimensions des pions 28 et des orifices 27 permet par ailleurs d'adapter indifféremment une tête d'une certaine dimension avec une tige de taille différente ce qui autorise une grande modularité.

D'autres moyens d'assemblage mécanique de la tête sur la tige sont bien-entendu possibles.

Les différences d'épaisseur entre les différentes bases peuvent aller de 0,5 mm à 1,5 mm par taille.

Les figures 12 à 16 montrent des tiges pour élément distal ou proximal de formes et de longueurs différentes selon des modes de réalisation de l'invention.

La tige 33 de la figure 12 présente d'un côté une plaque 34 anti-enfoncement du type décrit en référence de la figure 9, percée d'un orifice de réception du pion de la tête.

La partie pleine 35 est prolongée dans la direction opposée à la plaque d'une portion 36 en biseau plat munie d'une fente débouchante 37 sur toute sa longueur de faible épaisseur, par exemple 2 mm dans le plan dorsal palmaire, ce qui lui confère une forme normale de bec de canard.

La figure 13 montre un autre mode de réalisation de la tige 38 représentant une partie 39 propre à être enfoncée dans l'os, sensiblement pyramidale ou tronconique de section transversale en forme de H, libérant ainsi de part et d'autre du plan dorso-palmaire 40 des évidements sensiblement parallélépipédiques, de l'extrémité 41 en forme de bec à la plaque 42 anti-enfoncement.

La figure 14 montre une autre tige 43 comprenant une partie 44 de forme tronconique munie sur sa surface inférieure d'une goulotte 45 donnant à la tige une forme évidée.

La figure 15 montre quant à elle en coupe une demi-tige 46 en forme de tube 47 sensiblement cylindrique comprenant une portion d'extrémité 48 munie de la plaque 49, percée d'un évident 50 pour coopérer avec le pion de la tête comme décrit précédemment, l'évidement 50 étant prolongé d'un alésage 51 sur toute la longueur du tube 47.

La figure 16 montre quant à elle une tige 52 en forme de tôle pliée, présentant une partie d'extrémité 53 de section transversale en forme de U.

Dans les modes de réalisation de l'invention plus particulièrement décrits ici, les tiges sont rugueuses.

Cette rugosité est par exemple obtenue par sablage ou corindonage, ou par des cannelures transversales (non représentées), permettant d'augmenter l'adhérence dans l'os.

Afin d'améliorer encore l'ancrage un revêtement ostéo-intégrateur est avantageusement prévu par exemple du type Hydroxyapatite (initiales HAP) ou formé en titane micro-poreux par dépôt plasma.

On va maintenant décrire la mise en place d'une prothèse selon l'invention lors d'une opération d'implantation.

Après ouverture du doigt et section des parties osseuses endommagées de façon à mettre en place la prothèse, de façon connue en elle-même, (première étape de résection osseuse) on prépare les logements des tiges (introduction de râpes), puis on introduit dans le canal médullaire la tige de fixation.

On vérifie alors l'espace disponible pour la mise en place des têtes puis on emboîte la tête proximale condylienne 9 qui vient se fixer de façon précise et bloquée par le biais du cône morse sur ladite tige.

On effectue alors un test avec un fantôme de l'autre tête distale.

Puis, après le choix de l'épaisseur en fonction de la résection préalable de l'os, on met en place de l'autre côté, la tige 8 dans la phalange distale et l'articulation est finalisée par mise en place de la tête 10.

Le fait que la tête 10 puisse être choisie avec plusieurs épaisseurs différentes pour sa base, permet donc un ajustage pendant l'opération.

L'ensemble de ces opérations s'effectue de façon connue en elle-même à l'aide d'ancillaires dédiés.

Comme il va de soi et comme il résulte également de ce qui précède, la présente invention n'est pas limitée aux modes de réalisation plus particulièrement décrits. Elle en embrasse au contraire toutes les variantes et notamment celles où les deux têtes 9 et 10 sont en matériau souple, celle où les tiges sont en polyéthylène bio-compatible ou en autre métal que le titane, celles où la prothèse est une prothèse IP pouce ou gros orteil, ou une prothèse IPD, ce qui implique des dimensions différentes et un dessin des surfaces articulaires différent de façon à autoriser une mobilité latérale adéquate.

## Revendications

1. Implant orthopédique (2) utilisé en arthroplastie digitale comprenant un premier élément (3) pour phalange proximale (4) et un second élément (5) pour phalange adjacente distale (6), chaque élément comportant respectivement une tige d'implantation (7, 8) dans l'os et une tête d'articulation interphalangienne (9, 10), la tête (9) du premier élément présentant une surface(11) d'articulation condylienne, bi-convexe, comportant une vallée centrale (12), et la tête (10) du second élément présentant une surface (13) d'articulation bi-concave agencée pour coopérer avec ladite surface bi-convexe et comportant une crête centrale (14), **caractérisé en ce que** les surfaces (11, 13) au repos sont congruentes dans le plan frontal de l'articulation au niveau des condyles et non congruentes dans le plan sagittal.

2. Implant orthopédique selon la revendication 1, **caractérisé en ce que** les rayons de courbure (R1, R'1) des condyles (15, 16) de la surface bi-convexe du premier élément sont respectivement identiques aux rayons de courbure (R2, R'2) de la surface bi-concave (17,18) correspondante du second élément dans le plan frontal,
et **en ce que** le rayon de courbure (R3) de la surface proximale du premier élément est inférieur au rayon de courbure (R4) de la surface distale correspondante du second élément dans le plan sagittal.

3. Implant selon la revendication 2,
**caractérisé en ce que** les rayons de courbures sont tels que R1 = R'1 = R2 = R'2 .

4. Implant selon l'une quelconque des revendications précédentes, **caractérisé en ce que** les surfaces et la matière des têtes sont agencées pour coopérer à frottement sur une ligne de frottement dans le plan frontal en l'absence de pression longitudinale et sur une surface d'écrasement dans ledit plan frontal en cas de pression longitudinale.

5. Implant selon la revendication 4, **caractérisée en ce que** la matière de la surface condylienne (11) bi-convexe de la tête (10) du premier élément (9) est un matériaux polymère souple de module d'élasticité E ≤ 30 Gpa.

6. Implant selon la revendication 5 en ce que le module d'élasticité de la surface bi-convexe bi-condylienne est telle que E ≤ 2 Gpa.

7. Implant selon l'une quelconque des revendications précédentes, **caractérisé en ce que** les surfaces respectives des têtes des deux éléments placées en contact en vis à vis, définissent un espace inter-condylien (21) de volume non nul sur toute la longueur de la vallée centrale (12), permettant une lubrification améliorée de l'articulation entre les têtes.

8. Implant selon la revendication 7, **caractérisé en ce que**, dans le plan frontal, la vallée (12) de la tête bi-condylienne du premier élément présentant un rayon de courbure R5, et la crête (14) de la surface bi-concave du second élément présente un rayon de courbure R6 > R5.

9. Implant selon l'une quelconque des revendications précédentes, **caractérisé en ce que**, les tiges d'implantation (8, 9) ont une forme de pieu polygonal proportionné pour être reçu à l'intérieur de la cavité médullaire d'une phalange et muni d'une butée anti-enfoncement.

10. Implant selon l'une quelconque des revendications précédentes, **caractérisé en ce que** au moins une tige d'implantation (33, 38, 43, 46, 52) présente une forme au moins en partie ajourée dans le plan dorso-palmaire (40).

11. Implant selon la revendication 10, **caractérise en ce que** au moins une tige d'implantation (38, 52) présente une section transversale en H, en U ou en V.

12. Implant selon la revendication 10, **caractérise en ce que** au moins une tige d'implantation (33, 43, 46) présente une fente (37) en bec de canard, ou une forme évidée (44) en demi-cylindre côté palmaire ou en demi-tube (47).

13. Implant selon l'une quelconque des revendications précédentes, **caractérisé en ce que** les tiges d'implantation des éléments étant amovibles par rapport aux têtes correspondantes, elles comportent des moyens (26) de connexion avec lesdites têtes.

14. Implant selon la revendication 13, **caractérisé en ce que** les moyens de connexion sont formés par un emmanchement conique (27) d'un pion (28) solidaire de la tête dans un évidemment (28') ménagé dans la tige correspondante.

15. Implant selon la revendication 14, **caractérisé en ce que** l'emmanchement conique (27) est un cône Morse.

16. Implant selon l'une quelconque des revendications 13 et 14, **caractérisé en ce que** la surface bi-concave comporte une base (32, 33, 34) munie du pion de connexion, d'épaisseur déterminée.

17. Implant selon l'une quelconque des revendications précédentes, **caractérisée en ce que** les moyens (26) de connexion entre une tige et la tête correspondante comprennent de plus un évidemment ou une rainure (31) formé dans l'une des parties propre à recevoir une plaquette (30) de forme complémentaire solidaire de l'autre partie.

18. Implant selon l'une quelconque des revendications précédentes, **caractérisé en ce que** la surface bi-convexe d'articulation bi-condylienne et la surface bi-concave correspondante présentent des rayons de courbure uniformes dans le plan frontal et des rayons de courbure variables dans le plan sagittal.

19. Ensemble d'implants selon l'une quelconque des revendications précédentes, **caractérisé en ce que** les moyens de connexion sont identiques entre d'une part, plusieurs têtes de tailles différentes et d'autre part, des tiges identiques ou différentes de premier éléments et/ou de second éléments.

## Patentansprüche

1. Orthopädisches Implantat (2) für die Fingerarthroplastik mit einem ersten Element (3) für eine proximale Phalanx (4) und einem zweiten Element (5) für eine angrenzende distale Phalanx (6), wobei jedes Element jeweils einen Stift (7, 8) zum Implantieren in den Knochen und einen Interphalanxgelenkkopf (9, 10) aufweist, wobei der Kopf (9) des ersten Elements eine bikonvexe kondyläre Gelenkfläche (11) mit einem mittigen Tal (12) aufweist, und wobei der Kopf (10) des zweiten Elements eine bikonkave Gelenkfläche (13) aufweist, die angeordnet ist, um mit der bikonvexen Fläche zusammenzuwirken, und einen mittigen Kamm (14) aufweist,
**dadurch gekennzeichnet,**
**dass** die Flächen (11, 13) in Ruhestellung in der Frontebene des Gelenks an den Kondylen kongruent sind und in der Sagittalebene nicht kongruent sind.

2. Orthopädisches Implantat nach Anspruch 1,
**dadurch gekennzeichnet,**
**dass** die Biegeradien (R1, R'1) der Kondylen (15, 16) der bikonvexen Fläche des ersten Elements jeweils identisch sind mit den Biegeradien (R2, R'2) der entsprechenden bikonkaven Fläche (17, 18) des zweiten Elements in der Frontebene und dass der Biegeradius (R3) der proximalen Fläche des ersten Elements kleiner ist als der Biegeradius (R4) der entsprechenden distalen Fläche des zweiten Elements in der Sagittalebene.

3. Implantat nach Anspruch 2,
**dadurch gekennzeichnet,**
**dass** die Biegeradien derart sind, dass R1 = R'1 = R2 = R'2.

4. Implantat nach einem der vorhergehenden Ansprüche,
**dadurch gekennzeichnet,**
**dass** die Flächen und das Material der Köpfe angeordnet sind, um auf einer Reibungslinie in der Frontebene ohne Längsdruck und auf einer Quetschfläche in der Frontebene bei Längsdruck durch Reibung zusammenzuwirken.

5. Implantat nach Anspruch 4,
**dadurch gekennzeichnet,**
**dass** das Material der kondylären bikonvexen Fläche (11) des Kopfes (10) des ersten Elements (9) ein flexibles Polymermaterial mit einem Elastizitätsmodul E ≤ 30 Gpa ist.

6. Implantat nach Anspruch 5,
**dadurch gekennzeichnet,**
**dass** das Elastizitätsmodul der bikonvexen bikondylären Fläche derart ist, dass E ≤ 2 Gpa.

7. Implantat nach einem der vorhergehenden Ansprüche,
**dadurch gekennzeichnet,**
**dass** die jeweiligen Flächen der Köpfe der zwei Elemente, die sich berührend einander gegenüberliegend angeordnet sind, einen interkondylären Raum (21) begrenzen, dessen Rauminhalt auf der gesamten Länge des mittigen Tals (12) nicht gleich Null ist und der eine verbesserte Schmierung des Gelenks zwischen den Köpfen ermöglicht.

8. Implantat nach Anspruch 7,
**dadurch gekennzeichnet,**
**dass** in der Frontebene das Tal (12) des bikondylären Kopfes des ersten Elements einen Biegeradius R5 aufweist und der Kamm (14) der bikonkaven Fläche des zweiten Elements einen Biegeradius R6 > R5 aufweist.

9. Implantat nach einem der vorhergehenden Ansprüche,
**dadurch gekennzeichnet,**
**dass** die Implantationsstifte (8, 9) die Form eines vieleckigen Pfahls aufweisen, der dimensioniert ist, um in den medullären Hohlraum einer Phalanx aufgenommen zu werden, und mit einem Anti-Eindring-Anschlag versehen ist.

10. Implantat nach einem der vorhergehenden Ansprüche,
**dadurch gekennzeichnet,**
**dass** mindestens ein Implantationsstift (33, 38, 43, 46, 52) in der dorsalpalmaren Ebene (40) eine zumindest teilweise durchbrochene Form aufweist.

11. Implantat nach Anspruch 10,
**dadurch gekennzeichnet,**
**dass** mindestens ein Implantationsstift (38, 52) einen H-, U- oder V-förmigen Querschnitt aufweist.

12. Implantat nach Anspruch 10,
**dadurch gekennzeichnet,**
**dass** mindestens ein Implantationsstift (33, 43, 46) einen entenschnabelförmigen Schlitz (37) oder eine auf der Palmarseite halbzylindrisch ausgehöhlte Form (44) oder halbrohrförmig ausgehöhlte Form (47) aufweist.

13. Implantat nach einem der vorhergehenden Ansprüche,
**dadurch gekennzeichnet,**
**dass** die Implantationsstifte der Elemente, da sie in Bezug auf die entsprechenden Köpfe abnehmbar sind, Mittel (26) zum Verbinden mit den Köpfen aufweisen.

14. Implantat nach Anspruch 13,
**dadurch gekennzeichnet,**
**dass** die Verbindungsmittel durch ein konisches Einsteckende (27) eines mit dem Kopf fest verbundenen Zapfens (28) in einer Ausnehmung (28') gebildet sind, die in dem entsprechenden Stift ausgebildet ist.

15. Implantat nach Anspruch 14,
**dadurch gekennzeichnet,**
**dass** das konische Einsteckenende (27) ein Morse-Konus ist.

16. Implantat nach einem der Ansprüche 13 und 14,
**dadurch gekennzeichnet,**
**dass** die bikonkave Fläche eine Basis (32, 33, 34) aufweist, die mit dem eine bestimmte Dicke aufweisenden Verbindungszapfen versehen ist.

17. Implantat nach einem der vorhergehenden Ansprüche,
**dadurch gekennzeichnet,**
**dass** die Verbindungsmittel (26) zwischen einem Stift und dem entsprechenden Kopf zusätzlich eine Ausnehmung bzw. Nut (31) umfassen, die in einem der Bereiche ausgebildet ist, der geeignet ist, ein komplementär geformtes Plättchen (30) aufzunehmen, das mit dem anderen Bereich fest verbunden ist.

18. Implantat nach einem der vorhergehenden Ansprüche,
**dadurch gekennzeichnet,**
**dass** die bikonvexe bikondyläre Gelenkfläche und die entsprechende bikonkave Fläche einheitliche Biegeradien in der Frontalebene und variable Biegeradien in der Sagittalebene aufweisen.

19. Anordnung von Implantaten nach einem der vorhergehenden Ansprüche,
**dadurch gekennzeichnet,**
**dass** die Verbindungsmittel zwischen einerseits mehreren Köpfen unterschiedlicher Größe und andererseits identischen oder unterschiedlichen Stiften erster und/oder zweiter Elemente identisch sind.

## Claims

1. Orthopaedic implant (2) used in arthroplasty of the fingers including a first element (3) for a proximal phalange (4) and a second element (5) for a distal adjacent phalange (6), each element respectively comprising a rod (7, 8) for implantation in the bone and an interphalangeal joint head (9, 10), the head (9) of the first element having a bi-convex condylar joint surface (11) comprising a central valley (12), and the head (10) of the second element having a bi-concave joint surface (13) configured to co-operate with the said bi-convex surface and comprising a central ridge (14), **characterised in that** the surfaces (11, 13) at rest are congruent in the frontal plane of the joint at the condyles and non-congruent in the sagittal plane.

2. Orthopaedic implant according to claim 1, **characterised in that** the radii of curvature (R1, R2) of the condyles (15, 16) of the bi-convex surface of the first element are respectively identical to the radii of curvature (R2, R'2) of the corresponding bi-concave surface (17, 18) of the second element in the frontal plane,
and **in that** the radius of curvature (R3) of the proximal surface of the first element is less than the radius of curvature (R4) of the corresponding distal surface of the second element in the sagittal plane.

3. Implant according to claim 2, **characterised in that** the radii of curvature are such that R1 = R'1 = R2 = R'2.

4. Implant according to any one of the preceding claims, **characterised in that** the surfaces and the material of the heads are configured to co-operate by friction on a line of friction in the frontal plane in the absence of longitudinal pressure and on a "flattened" surface in the said frontal plane in the presence of longitudinal pressure.

5. Implant according to claim 4, **characterised in that** the material of the bi-convex condylar surface (11) of the head (10) of the first element (9) is a flexible polymer material with a modulus of elasticity E ≤ 30 Gpa.

6. Implant according to claim 5, **characterised in that** the modulus of elasticity of the bi-condylar bi-convex surface is such that E ≤ 2 Gpa.

7. Implant according to any one of the preceding claims, **characterised in that** the respective surfaces of the heads of the two elements placed in contact facing one another define an inter-condylar space (21) with a volume of other than zero over the entire length of the central valley (12), allowing improved lubrication of the joint between the heads.

8. Implant according to claim 7, **characterised in that** in the frontal plane the valley (12) of the bi-condylar head of the first element has a radius of curvature R5, and the ridge (14) of the bi-concave surface of the second element has a radius of curvature R6 > R5.

9. Implant according to any one of the preceding claims, **characterised in that** the implantation rods (8, 9) have a polygonal stake shape proportioned to be received inside the medullar cavity of a phalange and provided with an anti-sinkage stop.

10. Implant according to any one of the preceding claims, **characterised in that** at least one implantation rod (33, 38, 43, 46, 52) has an at least partially pierced shape in the dorso-palmar plane (40).

11. Implant according to claim 10, **characterised in that** at least one implantation rod (38, 52) has a transverse section in the shape of an H, a U or a V.

12. Implant according to claim 10, **characterised in that** at least one implantation rod (33, 43, 46) has a duckbill slot (37), or a semi-cylindrical recessed shape (44) on the palmar side or a semi-tubular recessed shape (47).

13. Implant according to any one of the preceding claims, **characterised in that** as the implantation rods of the elements are detachable in relation to the corresponding heads, they comprise means (26) for connection with the said heads.

14. Implant according to claim 13, **characterised in that** the means for connection are formed by tapering sleeving (27) of a projection (28) integral with the head in a recess (28') formed in the corresponding rod.

15. Implant according to claim 14, **characterised in that** the tapering sleeving (27) is a Morse taper.

16. Implant according to any one of claims 13 and 14, **characterised in that** the bi-concave surface comprises a base (32, 33, 34) provided with the connecting projection, of predetermined thickness.

17. Implant according to any one of the preceding claims, **characterised in that** the means for connection (26) between a rod and the corresponding head additionally include a recess or a groove (31) formed in one of the parts capable of receiving a plate (30) of complementary shape integral with the other part.

18. Implant according to any one of the preceding claims, **characterised in that** the bi-convex surface of the bi-condylar joint and the corresponding bi-concave surface have uniform radii of curvature in the frontal plane and variable radii of curvature in the sagittal plane.

19. Set of implants according to any one of the preceding claims, **characterised in that** the means for connection are identical between on one hand a plurality of heads of different sizes and on the other hand identical or different rods of first elements and/or second elements.
